Europäisches Patentamt

European Patent Office

Office européen des brevets

(19)

(11) Numéro de publication: **0 266 278 B1**

## (12) FASCICULE DE BREVET EUROPEEN

(45) Date de publication de fascicule du brevet: **23.12.92**

(51) Int. Cl.5: **G01N 33/546**, G01N 33/53, B01F 11/00

(21) Numéro de dépôt: **87420249.2**

(22) Date de dépôt: **24.09.87**

Demande divisionnaire 91101049.4 déposée le 24/09/87.

(54) **Procédé et dispositif de dosage de substances d'intérêt clinique réactives immunologiquement.**

(30) Priorité: **30.09.86 FR 8613782**

(43) Date de publication de la demande:
**04.05.88 Bulletin 88/18**

(45) Mention de la délivrance du brevet:
**23.12.92 Bulletin 92/52**

(84) Etats contractants désignés:
**BE CH DE ES FR GB GR IT LI LU NL SE**

(56) Documents cités:
EP-A- 0 005 978      EP-A- 0 094 216
EP-A- 0 106 122      EP-A- 0 163 312
EP-A- 0 174 195      EP-A- 0 186 946
EP-A- 0 189 389      FR-A- 1 409 780
FR-A- 1 596 064      GB-A- 2 040 441
GB-A- 2 136 565      US-A- 3 488 156
US-A- 4 203 724      US-A- 4 239 746
US-A- 4 250 394

(73) Titulaire: **INDICIA Société Civile d'Etudes et de Recherches (S.C.E.R.)**
**87 rue de la République**
**F-69600 Oullins(FR)**

(72) Inventeur: **Serres, Pierre-François**
**4, rue Etienne Radix**
**F-69630 Chaponost(FR)**

(74) Mandataire: **Nony, Michel et al**
**Cabinet NONY & CIE 29, rue Cambacérès**
**F-75008 Paris(FR)**

Rank Xerox (UK) Business Services

## Description

La présente invention concerne un procédé de dosage de substances d'intérêt clinique réactives immunologiquement. Elle concerne également un dispositif pour la mise en oeuvre de ce procédé.

Par substances réactives immunologiquement, on entend principalement toutes les antigènes (incluant les haptènes), et les anticorps (monoclonaux ou polyclonaux) obtenus par fusion cellulaire, ou immunisation naturelle ou provoquée.

Par substances d'intérêt clinique réactives immunologiquement, on entend principalement toutes les molécules antigéniques ou anticorps dont le dosage, dans un échantillon biologique d'origine humaine ou animale, peut présenter un intérêt dans le diagnostic médical, la recherche clinique ou le suivi d'un processus pathologique ou d'une thérapeutique mise en oeuvre.

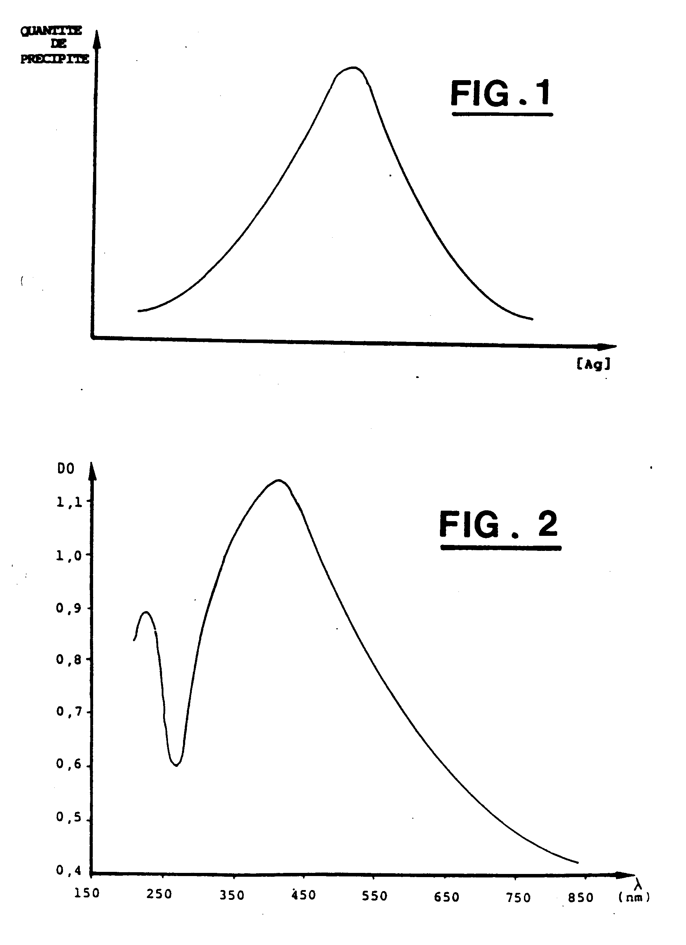

Le phénomène immunologique exploité dans ces procédés de dosage immunologique est représenté dans la figure 1.

Les procédés de dosage les plus couramment utilisés basés sur l'exploitation de la courbe de la figure 1 sont :
- l'immuno-diffusion radiale,
- la radio-immunologie, l'immuno-enzymologie, l'immuno-fluorescence,
- la néphélémétrie,
- d'autre part, depuis quelques années, quelques essais de versions quantitatives du "latex immuno essai", à savoir l'agglutination de microsphères synthétiques, ont été décrits. Jusque là, ces techniques largement employées et depuis longtemps (SINGER J.M., and PLOTZ C.M., the Latex fixation Test, American Journal of Medicine 21, 888 (1956)), à cause de leur simplicité et de leur faible coût ne présentaient qu'un aspect qualitatif (lame) ou semi-quantitatif (cupule).

Les formes qualitatives ou semi-quantitatives (lame-carte-cupule) du latex immuno-essai restent d'ailleurs, à l'heure actuelle, très employées dans le diagnostic clinique, et font l'objet de nombreux brevets et publications, en particulier dans le domaine de la virologie et microbiologie (KHABBAZ R.F., H.C. STANDFORD and Coll. 1985, Measurement of amikacin in serum by a Latex agglutination inhibition test, Journal of Clinical Microbiology, 22 : 699-701, brevet **US- 3 488 156,** demande de brevet **EP- 186 946).**

Dans ces techniques de tri ou de dépistage, l'antigène ou l'anticorps est fixé sur des microsphères synthétiques et l'absence ou la présence d'agglutination, par l'antigène ou l'anticorps correspondant est apprécié en tube, ou en cupule ou sur une lame de manière visuelle ou turbidimétrique, après agitation douce pour homogénéiser les mélanges des suspensions (ex : demandes **EP-A-0 106 122** et **EP-A-0 174 195).**

Les quelques essais de latex immuno-essai quantitatifs proposés dans la littérature (par exemple RIPOLL J. P., ROCH A.H., QUASH G A. and J. GRANGE, 1980, Journal of Immunological Methods 33, 159-173 demandes de brevet **EP 189 389** et **5978)** présentent essentiellement trois phases :

Phase I : Phase de fixation de l'antigène ou de l'anticorps sur une microsphère de polymère synthétique, de taille comprise entre 0,01 micromètre et 5 micromètres. La fixation est réalisée par tout moyen connu, identiques à ceux décrits dans les techniques sur lame, (par exemple : brevet américain **US-A-4 217 338).**

Phase II : Phase d'agglutination immunologique des microsphères synthétiques porteuses de l'antigène ou de l'anticorps. Cette phase II du procédé, à savoir l'agglutination immunologique des microsphères synthétiques, doit tendre vers deux buts :
- la réduction maximum de l'agglutination non spécifique des particules ; par agglutination non spécifique, on entend l'agglutination des particules due à des intéractions faibles (du type liaison hydrogène, ionique, ou Van der Waals), entre les microsphères ou les protéines fixées sur celles-ci.
- l'augmentation maximum de l'agglutination spécifique (immunologique) des particules.

Au cours de cette phase, des moyens essentiellement chimiques ou biochimiques sont mis en oeuvre pour stabiliser les particules, afin de minimiser l'agglutination non spécifique.

Pendant cette phase, des moyens sont également mis en oeuvre afin d'augmenter l'agglutination spécifique des particules, et donc la précision du dosage.

Pour réduire l'agglutination non spécifique des particules, le brevet américain **US-A-4 329 152** propose de les stabiliser par fixation d'albumine bovine rendue électro-négative à un pH sensiblement égal à 10. Le pH très basique, imposé de la sorte au réactif et aux gammes étalon est incompatible avec la survie des protéines, et an fait un réactif difficilement utilisable. D'autre part, la fiabilité du procédé en est réduite, car les solutions tampons à des pH si basiques sont très instables. D'autre part, l'albumine bovine fixée par liaison hydrophobe se détache progressivement des particules à pH 10, et perd progressivement sa capacité de stabilisation.

La stabilisation par l'albumine bovine à pH 8,2 est également utilisé dans le brevet **US-A-4 250 394** qui propose, de plus, une durée d'agitation traditionnelle très réduite.

Les agents chaotropiques utilisés quelquefois pour réduire l'agglutination non spécifique des particules présentent malheureusement l'inconvénient d'affaiblir ou de rompre les liaisons antigènes-anticorps.

D'autres procédés de stabilisation très compliqués et inutilisables dans un réactif industriel mettent en jeu des mélanges de particules légères et lourdes, (par exemple demande de brevet **EP-163 312**) et des centrifugations.

Pour favoriser l'agglutination spécifique des particules, c'est-à-dire l'agglutination immunologique, les procédés décrits utilisent une agitation traditionnelle, thermostatée dans un bain marie à 37°C ou 40°C, pendant des durées variant de 1/2 heure à une heure. De telles durées d'incubation à 37°C, outre qu'elles favorisent l'agglutination non spécifique indésirable des particules, imposent un chronométrage rigoureux et fastidieux dans l'introduction initiale du réactif dans chaque tube de la série, et dans le processus d'arrêt de la réaction de la série de tubes.

L'augmentation de l'agglutination spécifique (immunologique des particules), est quelquefois pratiquée en ajoutant, dans le milieu réactionnel, des additifs comme par exemple, de la dextrose connue sous la marque commerciale "Dextran" ou du polyéthylène glycol. L'inconvénient de ces substances est qu'elles sont souvent largement hydrophobes, et qu'elles se fixent donc sur les fragments Fc des anticorps fixés sur les microparticules, fragments eux-mêmes très hydrophobes, entraînant donc une agglutination non immunologique des particules, et induisant ainsi des interférences, et par la même des faux positifs aux réactions.

La demande EP-A-0106122 propose également une méthode de dosage d'immuno-gammaglobulines incluant une phase d'agglutination de l'anticorps avec' des particules de latex et une phase de mesure de l'agglutination. La réaction d'agglutination est contrôlée par une simple agitation de type circulaire et par des dilutions appropriées.

La demande EP-A-0174195 concerne une méthode d'agglutination d'anticorps et d'antigène en vue de détecter des antigènes ou des anticorps notamment. La réaction d'agglutination s'effectue de manière classique par utilisation d'un latex, chauffage pendant 30 minutes à 56°C, emploi d'albumine bovine et agitation traditionnelle.

Enfin, le brevet U.S.-A-4 250 394 propose de mesurer les propriétés de radiations électromagnétiques d'un échantillon, en vue de doser un composé à propriété immunitaire, du type anticorps. Le dosage implique une réaction d'agglutination impliquant un latex recouvert d'albumine bovine, et une phase d'agitation traditionnelle.

C'est l'un des buts de la présente invention de supprimer l'agglutination non spécifique des particules, et d'augmenter simultanément l'agglutination immunologique spécifique des particules, sans présenter tous les inconvénients décrits ci-dessus, et de surcroît, en diminuant considérablement la durée de l'opération.

Phase III : Phase de lecture du résultat

Actuellement, les techniques de lecture du résultat de l'agglutination dans les latex-immuno essais quantitatifs sont :

- le comptage de particules,
- l'opacimétrie, encore appelée turbidimétrie (pratiquée quelquefois dans le visible ou l'infra-rouge, le plus souvent dans le proche infra-rouge),
- la néphélémétrie laser,
- l'analyse centrifuge.

La néphélémétrie laser mesure la lumière diffusée par les agrégats de particules de latex. Cette technique décrite par exemple dans l'article suivant : GRANGE J., ROCH A.M., and G.A. QUASH, 1977, Journal of Immunogical Methods, 18, 326-375, présente l'inconvénient de nécessiter un appareillage de lecture coûteux et sophistiqué.

Le comptage de particules, décrit par exemple dans l'article suivant : MAGNUSSON C.G., MASSON P.L., Journal of Allergy Clin. and Immunol., 70:326, 1982, nécessite également un appareillage complexe et coûteux, à la portée de peu de laboratoires.

L'opacimétrie dans le visible, également dénommée turbidimétrie dans le visible, mesure la lumière transmise par la suspension de particules, c'est-A-dire la lumière qui n'est ni absorbée par les particules, ni diffusée par celles-ci. L'opacimétrie ou turbidimétrie des latex immuno-essais, qualitatifs ou quantitatifs, doit être pratiquée à une longueur d'onde assez proche de la taille des particules. La validité de la mesure turbidimétrique impose des suspensions très diluées, afin de pouvoir visualiser l'effet écran souhaité et, comme il est indiqué dans le Certificat d'Addition no 78 28250 du brevet français no 77 25049, afin de minimiser l'absorption de lumière par les particules.

Les mesures turbidimétriques (ou opacimétriques) dans le visible des latex immuno-essais décrites dans la littérature (exemple : BERNARD A.M., LAUWERYS R.R., 1982 Clinica Chemica Acta 119, 335-339) utilisent, selon la taille de la particule, différentes longueurs d'ondes du visible, par exemple 360, 400, 450 et beaucoup plus qénéralement, entre 600 et 750 nanomètres. La précision des résultats turbidimétrique est médiocre, car les suspensions de particules doivent être très diluées, afin, comme on l'a dit, d'obtenir les variations d'effet-écran soubhaitées, et afin de minimiser l'absorption des latex. D'ailleurs, il faut généralement dans ces techniques, pour augmenter la précision du résultat, utiliser des cuves de lecture dont le chemin optique est important, de l'ordre de deux à quatre centimètres.

Afin d'utiliser des suspensions de latex plus concentrées, et d'augmenter ainsi la précision des dosages, et afin de na pas être gêné par l'absorption des latex dans le visible ou dans l'ultra-violet, le brevet français **FR-A-77/25049** et son certificat d'addition **FR-A-78/28250,** préconisent,pour la lecture des particules, l'opacimétrie infra-rouge. En effet, les latex n'absorbent plus la lumière dans l'infra-rouge.

Le brevet **US-A-4 203 724** propose, de la même façon, de lire le résultat de l'agglutination des microparticules dans la zone infra-rouge ou infra-rouge lointain, afin de ne pas être gêné par l'absorption vraie des microparticules qui a lieu dans la zone ultra-violet ou visible. Dans ce procédé, il est pratiqué une opacimétrie infra-rouge des agrégats desdites particules mettant en oeuvre un opacimètre infra-rouge qui représente, comme déjà dit, un appareillage coûteux et sophistiqué.

C'est un autre but de la présente invention de proposer un procédé de lecture simple et nouveau du résultat de l'agglutination spécifique (immunologique) des microparticules.

Le but essentiel de la présente invention est donc de remédier aux nombreux inconvénients (des phases II et III) soulignés précédemment, par la mise en oeuvre d'un procédé et d'un appareillage d'agglutination simple et nouveau, et par la mise en oeuvre d'un procédé de lecture du résultat également simple et nouveau, faisant ainsi de ce type de dosage un procédé très simple de mise en oeuvre, très rapide et à la portée de n'importe quel laboratoire.

Le procédé selon l'invention de dosage de substances d'intérêt clinique réactives immunologiquement est donc du type dans lequel :
- on greffe préalablement une substance active immunologiquement, jouant le rôle de réactif doseur, sur des microparticules,
- on agglutine lesdites microparticules par la substance d'intérêt clinique réactive immunologiquement,
- on mesure par lecture le résultat issu de la réaction d'agglutination, que l'on compare à une gamme étalon.

Le procédé de dosage selon l'invention se caractérise en ce que la phase d'agglutination s'effectue en soumettant lesdites microparticules greffées à un mouvement périodique de fréquence comprise entre 4 et 40 Hertz, et de préférence 20 Hertz, et d'amplitude variant de quelques millimètres à quelques centimètres, de préférence de l'ordre de 4 millimètres.

En effet, on a constaté que si la fréquence était inférieure à 4 Hertz, cela diminuait l'intérêt de la méthode du fait que l'on augmente la durée de la phase d'agglutination afin d'obtenir un résultat utilisable, et, du fait que l'effet recherché sur l'agglutination non spécifique devient médiocre ; par contre, si la fréquence est supérieure à 40 Hertz, on provoque la rupture des aggrégats (Immuns complexes) formés par les microparticules agglutinées.

Comme il apparait dans la description qui précède, dans la phase d'agglutination (phase II du procédé), afin de remédier à tous les inconvénients des procédés chimiques décrits ci-dessus visant à diminuer l'agglutination non spécifique des particules, ou augmenter l'agglutination immunologique de celles-ci, le moyen d'agglutination mis en oeuvre selon l'invention utilise un procédé mécanique qui atteint simultanément ces deux buts.

Ce procédé mécanique est basé sur l'observation que l'agglutination non spécifique, qui est liée à des interactions faibles entre les particules (liaisons de type ionique, hydrogène, Van der Waals) est rendue impossible ou est fortement inhibée par un mouvement périodique imprimé à celles-ci, de très faible amplitude et de très grande fréquence, quelle que soit la trajectoire du mouvement périodique (par exemple et commodément rectiligne, circulaire ou ovalisée). On a également montré qu'un tel procédé favorise notablement l'agglutination spécifique (immunologique) des microparticules porteuses de l'antigène ou de l'anticorps correspondant, et donc permet une réaction spécifique rapide et effectuée de surcroit à température ambiante.

La présente invention a également pour objet un procédé de dosage de substances d'intérêt clinique réactives immunlogiquement du type dans lequel :
- on greffe préalablement une substance active immunlogiquement, jouant le rôle de réactif doseur, sur des microparticules,
- on agglutine les dites microparticules par la substance d'intérêt clinique réactive immunologiquement,
- on mesure par lecture le résultat issu de la réaction d'agglutination, que l'on compare à une gamme étalon, caractérisé en ce que la phase de lecture du résultat s'effectue de la façon suivante:

- tout d'abord, on détermine, en en faisant le spectre d'absorption ultra-violet visible, le maximum d'absorption par les particules de la lumière ultra-violet visible d'une suspension en milieu liquide de microparticules non agglutinées, la longueur d'onde correspondant audit maximum d'absorption étant appelée λmax

- puis on procède, après la réaction d'agglutination immunologique, et après une dilution appropriée, à la lecture du résultat par spectrophotométrie d'absorption ultra-violet visible, en se plaçant au voisinage de ladite longueur d'onde λmax précédemment déterminée.

Il est surprenant de pouvoir procéder, notamment lorsque les microparticules sont des latex, à cette phase de lecture en utilisant l'absorption ultra-violet visible des particules de latex, étant donné que le certificat d'addition précédemment cité FR-A-78/28250 décrit ce phénomène comme un inconvénient majeur.

Il semble bien que dis le procédé de lecture selon l'invention, c'est la véritable absorption de la lumière ultra-violet- visible par les particules elles-mêmes qui est exploitée, alors que les méthodes turbidimétriques (opacimétriques) ne mesurent qu'une "absorption apparente" de la suspension des particules, c'est-à-dire, justement la lumière qui n'est pas absorbée par les particules.

En effet, on a observé, et c'est l'une des caractéristiques de l'invention, qu'une microparticule capable d'absorber la lumière ultra-violet-visible, dont la taille peut varier entre 0,01 et 5 micromètres de polymère synthétique présente, et ce, quelles que soient sa taille et sa nature chimique (par exemple : polystyrène, polyvinyl-toluène, acrylonitrile, styrène-butadiène, copolymères acrylontirile - acide acrylique, ester-acrylique, polyvinyl-butadiène), un maximum caractéristique d'absorption de lumière ultra-violet-visible avec un coefficient d'extinction qui dépend de la longueur d'onde. De plus, on a montré, et cette observation est utilisée dis le procédé de lecture selon l'invention, que si on effectue une mesure d'une suspension aqueuse de ces microparticules non agglutinées, par spectrophotométrie d'absorption à la longueur d'onde λmax correspondant au maximum d'absorption de lumière ultra-violet-visible des microparticules non agglutinées (λmax étant caractéristique d'une microparticule donnée), le résultat n'est pas du tout, ou très faiblement influencé par la présence dans le milieu d'agrégats (ou agglutinats) de ces mêmes microparticules car les agrégats de ces microparticules, quelle que soit leur taille, n'absorbent plus la lumière ultra-violet-visible à ce λmax, leurs maxima d'absorption étant très éloignés du λmax défini précédemment.

D'autre part, on a montré qu'une mesure par spectrophotométrie d'absorption ultra-violet-visible d'une suspension aqueuse desdites microparticules non agglutinées (monomères) obéit à la loi de Beer-Lambert, et cela même pour des concentrations en particules très élevées, ce qui en fait un net avantage par rapport au principe des mesures turbidimétriques, qui impose des suspensions très diluées.

Avantageusement, en pratique :

- la trajectoire du mouvement périodique peut être de toute nature, mais de préférence circulaire, rectiligne, ou ovalisée,
- la phase d'agglutination s'effectue à température ambiante,
- la durée du mouvement périodique imposée aux microparticules, durant la phase d'agglutination, est fonction de la fréquence de la vibration, de la trajectoire et de l'amplitude du mouvement. Elle est avantageusement de 10 minutes pour une fréquence de 20 Hertz et un mouvement circulaire périodique de 4 millimètres de diamètre,
- les microparticules, aptes à la lecture spectrophotométrique d'absorption ultra-violet-visible, sont constituées de matériaux naturels ou synthétiques, de toute nature absorbante, notamment de polystyrène, polyvinyl-toluène, acrylonitrile, styrène-butadiène, copolymères acrylonitrile - acide acrylique, ester acrylique, poly-vinylbutadiène, et de préférence de polystyrène,
- les microparticules sont de forme quelconque mais de préférence sous forme de microsphères,
- le diamètre des microsphères naturelles ou synthétiques est compris entre 0,01 et 5 micromètres, et de préférence voisin de 0,8 micromètre,
- les microparticules sont constituées d'un matériau absorbant mais non coloré,
- il est possible d'utiliser des microparticules absorbantes qui sont colorées ; dans ce cas la lecture du résultat par spectrophotométrie d'absorption ultra-violet-visible est effectuée soit à longueur d'onde correspondant à l'absorption maximum de la matière de la particule non agglutinée (λmax), soit à la longueur d'onde correspondant à l'absorption maximum de la couleur de la particule considérée.

L'invention concerne également un dispositif pour la mise en oeuvre du procédé. Ce dispositif est caractérisé en ce qu'il comporte au moins :

- une première série de tubes renfermant au moins une gamme étalon lyophilisée de la substance à doser,
- une seconde série de tubes renfermant une substance active immunologiquement et jouant le rôle de doseur, greffée sur des microparticules, la suspension appelée "réactif" étant en phase liquide, la concentration en poids des microparticules étant comprise entre 0,1 et 10 pour cent,
- une troisième série de tubes renfermant un échantillon lyophilisé de la solution de dilution de la gamme étalon.

La manière dont l'invention peut être réalisée et les avantages qui en découlent ressortiront mieux de l'exemple de réalisation qui suit, donné à titre indicatif et non limitatif, à l'appui des figures annexées.

La figure 1 est un diagramme représentatif du phénomène immunologique exploité.

La figure 2 est un diagramme repésentatif du spectre d'absorption ultra-violet-visible d'une suspension aqueuse de microsphères (non agglutinées) de polystyrène (non coloré) de 0,8 micromètre de diamètre.

La figure 3 est un diagramme représentatif des spectres d'absorption ultra-violet-visible de microsphères de polystyrène anticorps (non coloré) de 0,8 micromètre de diamètre non agglutinées (courbe I), et agglutinées par des concentrations croissantes de 50 nanogrammes/millilitre (courbe II), 100 nanogrammes/millilitre (courbe III), 500 nanogrammes/millilitre (courbe IV) et 1 microgramme/millilitre (courbe V) de l'antigène correspondant.

La figure 4 est un diagramme représentatif d'un dosage d'Immuno-gammaglobulines G humaines par le procédé selon l'invention.

La figure 5 est un diagramme illustrant les spectres d'absorption ultra-violet-visible d'une suspension aqueuse de microsphères de polystyrène rouge de 0,8 micromètre de diamètre.

La figure 6 est un diagramme illustrant les spectres d'absorption ultra-violet visible d'une suspension aqueuse de microsphères de polystyrène non colorées d'une part et jaunes d'autre part de 0,8 micromètre de diamètre ; la concentration en microsphères est la même dans les deux cas.

La figure 7 est une vue du boitier compartimenté relatif au procédé de mise en oeuvre de l'invention.

L'exemple qui suit correspond au dosage d'antigènes au moyen d'anticorps greffés sur des microsphères. Il est bien entendu que l'on pourrait tout aussi bien procéder au dosage d'anticorps au moyen d'antigènes , en suivant le même processus mais en remplaçant les antigènes par les anticorps et réciproquement.

Avant le dosage proprement dit, on procède tout d'abord au greffage des anticorps sur des microsphères synthétiques calibrées, dénommées également latex. Ceux-ci sont réalisés par exemple en polystyrène, polyvinyl-toluène, acrylonitrile, styrène-butadiène, copolymère acrylonitrile-acide acrylique, ester acrylique et polyvinyl butadiène. Les particules de latex K 140, K 109, K 610, Ψ512, Ψ513, Ψ480, Ψ502 (-COOH) ou Ψ169, Ψ181 (- CONH2) vendus sous ces dénominations commerciales par RHONE-POULENC, conviennent par exemple très bien au procédé.

Le greffage des anticorps sur les microsphères synthétiques s'effectue de manière connue, c'est-à-dire par fixation ionique, hydrophobe ou covalente.

Lorsque l'on fait appel à des accrochages de type ionique ou hydrophobe d'anticorps sur des microsphères de latex, ces accrochages qui se font de manière connue par simple contact de la protéine et du support, conduisent à des phénomènes de détachement (également dénommés relargages) des protéines du support. L'une des raisons des phénomènes de relargage est qu'il existe des échanges permanents entre la population d'anticorps du type immuno-gammaglobulines G ainsi fixée et la population desdites immunogammaglobulines G fréquemment présente dans l'échantillon à analyser.

Avantageusement, les méthodes de fixation covalente sont plus fiables et préférables dans ce type de procédé de dosage immunologique. Les méthodes de fixation covalente d'anticorps ou d'antigène sur des microsphères synthétiques, ou latex, sont bien connues, de sorte qu'il n'y a pas lieu de les décrire ici plus en détail.

Dans le procédé selon l'invention, lorsque l'on met en jeu une fixation du type covalente, on fait appel avantageusement à un procédé de fixation qui n'a jamais été décrit dans ce type d'application et qui fournit de bons résultats.

Selon ce procédé de couplage, les radicaux fonctionnels présents sur les microsphères et mobilisés sont principalement les suivants :
- COOH, - CONH2, - CONH - NH2, - COOCH3.

Ces radicaux sont transformés en acyl-azotures (-CON3) par le procédé qui va être décrit, l'azoture réagissant avec des radicaux -NH2, -SH ou -OH des molécules biologiques (antigènes ou anticorps ) que l'on veut greffer.

Par exemple, les latex Ψ169 et Ψ181 sont traités tout d'abord à l'hydrazine en solution puis ensuite aux vapeurs de nitrite acide de sodium, dans des conditions douces, avant d'être mis en contact avec un mélange poly-L-lysine-anticorps. Les latex carboxylés Ψ480 ou Ψ502 sont carboxyméthylés au méthanol-acide, puis traités à l'hydrazine en solution, puis aux vapeurs de nitrite-acide de sodium dans des conditions douce s, avant d'être greffés dans un rapport approprié avec un mélange poly-L-lysine anticorps.

Exemple :

Greffage d'anticorps anti-immuno-gammaglobulines g humaines, et anti $\beta$ 2 micro-globuline humaine sur microsphères de polystyrène Ψ480 et Ψ502 (RHONE-POULENC) de diamètre respectif 0,8 et 0,85 micromètre.

Ce greffage se déroule suivant différentes phases résumées ci-dessous :
- méthylation des groupments carboxylés de latex :
  l'estérification des carboxyles libres des latex s'effectue dans un bain de méthanol acidifié, trois jours minimum, dix jours maximum, le plateau de saturation se trouvant en général à une moyenne de cinq jours,
- rinçage aqueux énergique,
- traitement des latex carboxyméthylés à l'hydrazine pendant 1 à 15 heures avec agitation à 20°C,
- rinçage aqueux énergique, à 0°C,
- traitement aux vapeurs de nitrite de sodium acidifié par de l'acide chlorhydrique (conditions douces) ; les constituants du mélange et le mélange étant réalisés extemporanément, et la réaction étant effectuée à 0°C,
- rinçage énergique avec tampon de couplage,
- greffage du mélange anticorps-poly-L-lysine ; la poly-L-lysine ainsi couplée aux latex-anticorps a un effet de saturation des sites de greffage (-CON3), et stabilise stériquement le réactif lors de sa conservation ; les proportions anticorps-poly-L-lysine varient selon l'enrichissement désiré en anticorps sur la microsphère synthétique, et selon que l'on désire utiliser la partie droite ou gauche par rapport au point d'équivalence de la courbe représentée au sein de la figure 1 ; il est à noter que le procédé selon l'invention permet l'utilisation aussi bien de la partie droite que de la partie gauche de ladite courbe,
- désorption des anticorps fixés par liaison ionique (KCl 3 M, pendant 15 minutes), et par liaison hydrophobe (KSCN 1M, pendant 10 minutes),
- rinçage
- stockage

Il va de soi que si les groupes fonctionnels des particules sont - CONH2 (exemple Ψ169 et Ψ181) ou -COOCH3, la phase à l'hydrazine représente la première phase du procédé de greffage.

La phase suivante correspond à la phase d'agglutination des microsphères, qui peut être effectuée de la façon suivante. On soumet les microsphères de latex à un mouvement circulaire périodique de fréquence égale à 20 Hertz et d'amplitude de 4 mm, et ce pendant une durée de dix minutes. Cette phase s'effectue à température ambiante, d'où une diminution notable de l'appareillage (pas de bain marie ou de tout autre type d'appareillage de chauffage). Cette phase d'agglutination ainsi réalisée permet, une forte diminution de l'agglutination non spécifique des particules. En effect, son efficacité sur l'agglutination non spécifique des particules est très grande : l'agglutination non spécifique lorsque l'on utilise l'un des procédés prédédemment décrits, c'est-à-dire à 37°C, pendant 1/2 heure au bain marie avec une agitation traditionnelle, est de l'ordre de 30 à 40%. L'agglutination non spécifique selon le procédé décrit dans l'invention varie de 2 à 5% seulement.

De plus, ce procédé ne nécessite pas de stabilisation de particules par de l'albumine bovine ou tout autre agent ionique rendu électro-négatif ou électro-positif à des pH très basiques, ou très acides, incompatibles avec la conservation des réactifs.

Le procédé permet donc par voie de conséquence, le maintien du réactif et des gammes étalon à des pH physiologiques (voisin de 7), assurant donc une très bonne intégrité et une excellente conservation des constituants. Il est à noter qu'il existe un autre avantage en évitant l'utilisation de substances stabilisatrices protéiques, comme le permet le procédé. En effet, les substances stabilisatrices protéiques, telles que l'albumine, sont source de contamination bactérienne, ce qui est très gênant dans un réactif industriel devant être conservé plusieurs mois.

De plus, le procédé ci-dessus permet simultanément avec une diminution très nette de l'agglutination non spécifique des particules, une augmentation notable de l'agglutination spécifique immunologique des particules.

Un plateau de saturation dans l'agglutination immunologique des microsphères est atteint rapidement en dix minutes environ. Une durée d'incubation si courte, outre qu'elle soit très intéressante pour l'utilisateur qui a un résultat de dosage extrêmement rapide, n'implique, d'autre part, pas de chronométrage particulièrement fastidieux, dans l'introduction initiale du réactif ou dans la phase d'arrêt de la réaction.
De plus, l'efficacité du procédé selon l'invention sur l'agglutination spécifique est telle que l'agitation peut être effectuée à température ambiante, ce qui évite comme déjà dit précédemment, l'utilisation d'appareillages chauffants ou de bains-marie, d'où un avantage certain et appréciable de commodité.

Selon l'invention, on a donc vérifié expérimentalement que l'agglutination non spécifique, qui est liée à des interactions faibles entre les particules (liaison de type ionique, hydrogène, Van der Waals) est rendue impossible ou fortement inhibée par un mouvement périodique imprimé à celle-ci, de très faible amplitude et de très grande fréquence, quelle que soit la trajectoire du mouvement périodique (par exemple et commodément rectiligne, circulaire ou ovalisée). On a également vérifié qu'un tel procédé favorise notablement l'agglutination spécifique (immunologique) des microsphères synthétiques porteuses de l'antigène ou de l'anticorps, en présence de l'antigène ou de l'anticorps correspondant, et donc permet une réaction spécifique rapide et effectuée à température ambiante.

La phase d'agglutination étant réalisée, on provoque une dilution aqueuse du milieu d'incubation contenant lesdites microsphères jusqu'à un volume de 1 à 4 millilitres. La dilution peut être effectuée avec de l'eau, une solution saline, ou tout autre solution tampon convenable. Le volume de dilution correspond à celui d'une cuve traditionnelle de spectrophotométrie de dimension 1 X 1 cm.

On procède alors à la phase de lecture et de mesure. En fait, antérieurement à la phase d'agglutination proprement dite, on réalise le spectre d'absorption dans l'ultra-violet-visible d'une suspension aqueuse des microsphères de polystyrène non coloré non agglutinées afin de déterminer le maximum d'absorption par les particules de la lumière ultra-violet-visible ; soit λmax la longueur d'onde représentant ce maximum d'absorption. On a montré que le maximum d'absorption λmax, caractéristique d'une microsphère naturelle ou synthétique donnée est identique, que ladite microsphère soit ou non revêtue d'une molécule anticorps ou antigène. A titre d'exemple, la figure 2 représente le maximum d'absorption dans la zone ultra-violet-visible d'une suspension concentrée de microsphères de polystyrène non coloré d'un diamètre de 0,8 micromètre, le spectre étant identique, lorsque les microsphères sont revêtues ou non d'un anticorps ou d'un antigène.

Il faut noter que la loi de Mie (intensité de la lumière diffusée vers l'avant) provoque un léger abaissement du pic d'absorption car la diffusion de la lumière vers l'avant est un phénomène inverse du phénomène d'absorption.

La longueur d'onde λmax ainsi déterminée, on procède après la phase d'agglutination immunologique, à la lecture du résultat par spectrophotométrie d'absorption ultra-violet-visible en se plaçant au voisinage de ladite longueur d'onde λmax précédemment déterminée. Dans l'exemple décrit, λmax a une valeur de 380 nanomètres.

L'expérience montre que les résultats ainsi obtenus selon l'invention, par spectrophotométrie d'absorption ultra-violet-visible, indiquent uniquement l'absorption des monomètres des microsphères, l'amplitude du pic (degré d'absorption, densité optique, pourcentage de transmission) étant proportionnelle à la concentration en microsphères monomères da ns le milieu (loi de Beer-Lambert).

Or, il est par ailleurs connu dans ce type de dosage que, une fois la réaction d'agglutination immunologique achevée, la concentration en momonères résiduels non agglutinés est proportionnelle au logarithme de la concentration initiale en antigène dans le milieu.

Le résultat de la lecture de la réaction par spectrophotométrie d'absorption ultra-violet-visible au voisinage du λmax d'absorption des monomères des microsphères non agglutinées, est donc ainsi proportionnel au logarithme de la concentration initiale en antigène dans le milieu.

Il est à noter que ce procédé est utilisable quelle que soit la nature du polymère (notamment ceux précisés précédemment), quelle que soit la taille des particules et leur coloration. Lorsque les particules de latex sont colorées, on peut se placer pour la lecture soit au λmax de la particule, soit au λmax de la couleur de celle-ci. En effet, dans le cas des latex colorés, les spectres d'absorption ultra-violet-visible présentent deux pics, l'un correspondant à la matière (indépendante de la couleur), l'autre correspondant au colorant de cette matière.

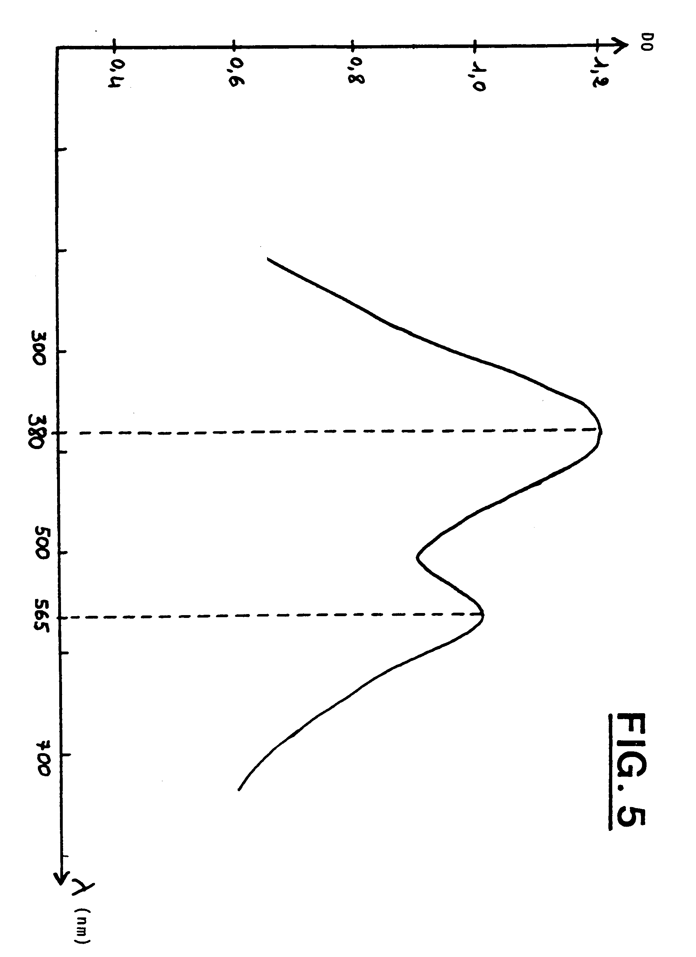

Ceci est illustré sur la figure 5 qui montre que le spectre d'absorption de lumière ultra-violet-visible par des microparticules de polystyrène rouge présente deux pics de maximum d'absorption. Le premier pic, à 380 nm correspond au maximum d'absorption de la substance des microparticules. Le second pic, à 560 nm pour des microparticules rouges, correspond au maximum d'absorption de la couleur de cette substance.

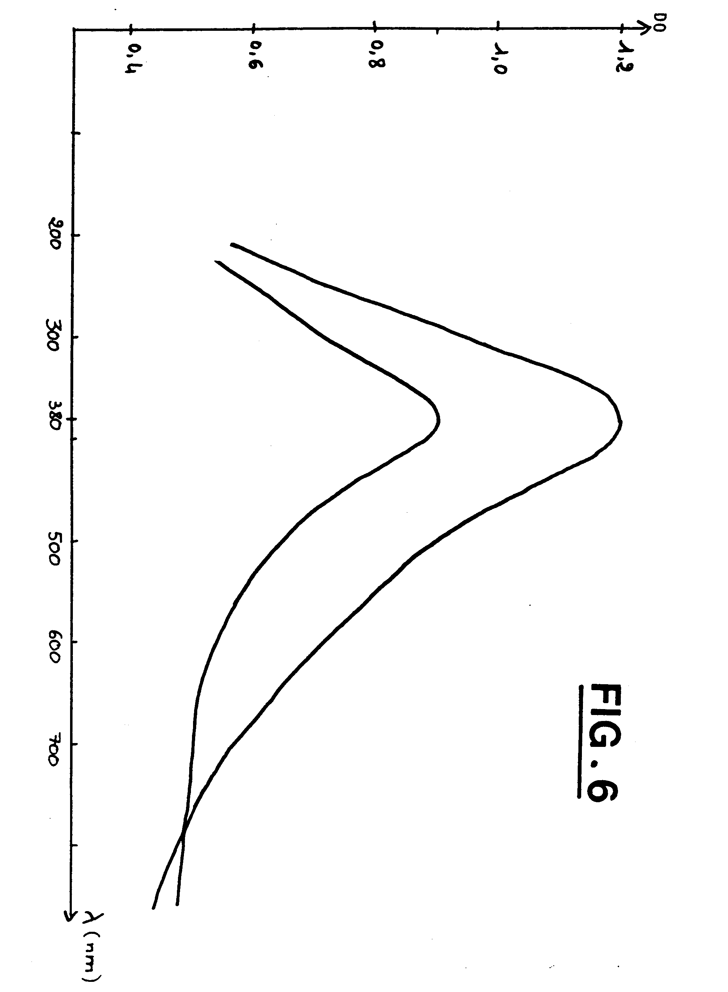

Ce phénomène peut être utilisé pour obtenir un effet de synergie entre les deux maxima d'absorption ainsi que cela est représenté à la figure 6. En effet sur ce diagramme, sont représentés deux spectres d'absorption de la lumière ultra-violet visible par des microparticules de polystyrène de 0,8 micromètres de diamètre. Le premier spectre représente l'absorption de microparticules non colorées alors que le second spectre représente l'absorption de microparticules colorées en jaune. La concentration en microparticules est la même dans les deux cas.

On observe que le premier spectre présente un pic à 380 nm qui est la longueur d'onde du maximum

8

d'absorption de la substance des microparticules.

Le second spectre présente également un pic à 380 nm, mais d'amplitude plus importante.

Or, on sait que la longueur d'onde du maximum d'absorption de la couleur jaune est environ 380 nm. Ainsi, dans ce cas, on a un effet de synergie entre le maximum d'absorption de la substance et le maximum d'absorption de la couleur des microparticules.

Cet effet de synergie permet, pour une même concentration en microparticules, d'augmenter la précision du dosage.

Il va de soi, dans la mise en oeuvre pratique du procédé, qu'on a intérêt à choisir une microsphère de polymère synthétique dont la nature, la taille ou la couleur présente un maximum d'absorption ultra-violet-visible à des longueurs d'onde qui n'interfèrent pas avec des longueurs gênantes, en particulier à 280 nanomètres correspondant à l'absorption des protéines. Avantageusement, le procédé selon l'invention utilise par exemple des microsphères carboxylées de polystyrène non coloré d'un diamètre de l'ordre de 0,8 micromètre, dont le maximum d'absorption ultra-violet-visible est situé comme déjà dit vers 380 nanomètres.

Cette longueur d'onde n'interfère pas avec le maximum d'absorption des protéines situé à 280 nanomètres. Cette longueur d'onde de 380 nanomètres peut être sélectionnée par n'importe quel spectrophotomètre ou photomètre, en utilisant indifféremment une lampe ultra-violet ou visible, compte tenu que la valeur de 380 nanomètres se situe à la frontière de l'ultraviolet et du visible. De plus, la lecture à 380 nanomètres en visible est à la portée de n'importe quel appareillage photométrique ou spectrophotométrique de laboratoire, d'où une simplification importante de l'appareillage nécessai re au procédé de dosage.

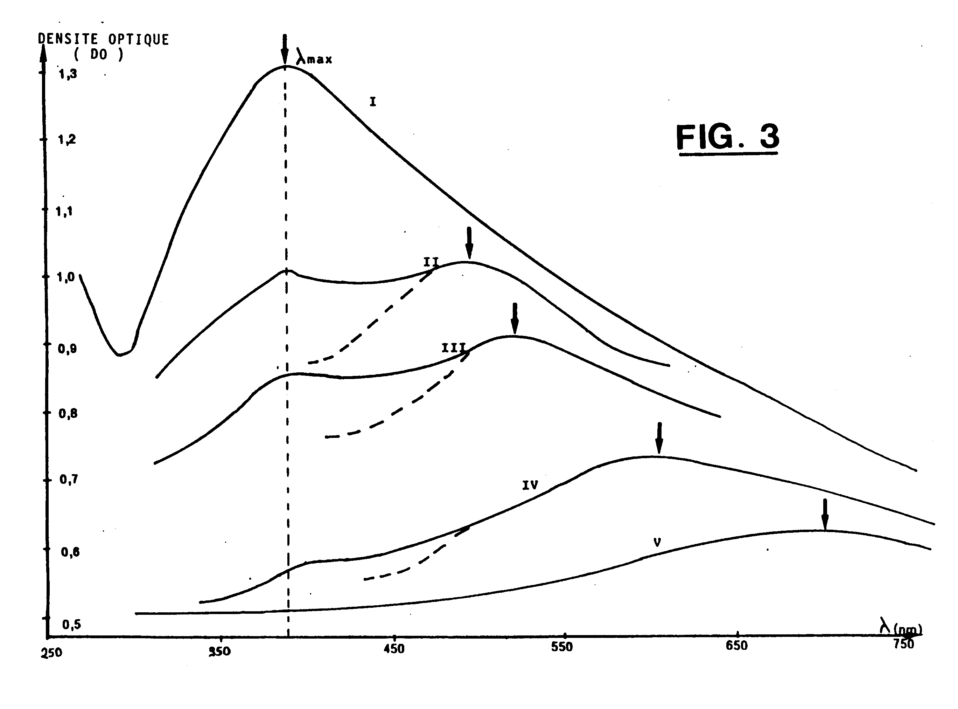

D'autre part, l'expérience montre que le maximum d'absorption λmax est caractéristique des microsphères désagglutinées, et que des agglutinats de ces mêmes microsphères, même lorsqu'ils sont de très faible taille, n'absorbent plus à ce maximum. En effet, on a observé que les agrégats des microsphères synthétiques de polymères ont un maximum d'absorption caractéristique de leur taille. On a également observé que les agglutinants ont un maximum d'absorption situé d'autant plus vers les grandes longueurs d'onde que leur taille est importante. La figure 3 indique (flèches) le maximum d'absorption des microsphères non colorées de polystyrène, greffées d'anticorps,

de 0,8 micromètre de diamètre, non agglutinées et les maxima d'absorption des agglutinats de taille croissante de ces mêmes microsphères. On observe :

- d'une part, l'absorption des microsphères résiduelles non agglutinées par l'antigène (à λmax),
- d'autre part, l'absorption des agrégats de taille croissante, induits par des concentrations croissantes en antigène.

Il ressort clairement de ce qui précède, qui si, par spectrophotométrie d'absorption ultra-violet-visible, en se plaçant à une longueur d'onde qui correspond au λmax d'absorption des microsphères non agglutinées (monomères), on effectue la lecture d'une solution contenant un mélange de microsphères monomères et d'agglutinats de celles-ci, la mesure ainsi effectuée ne concernera et donc ne détectera que les monomères des particules, car elles seules absorbent à ce maximum.

Ce mode de lecture nouveau, selon l'invention, présente par rapport à l'état de la technique précédemment décrit de nombreux avantages :

- il ne nécessite pas l'utilisation d'appareillages très sophistiqués et coûteux, tels que les compteurs de particules, les néphélémètres laser, les opacimètres infra-rouge,
- à l'inverse des techniques turbidimétriques dans le visible, il peut être utilisé avec des concentrations très fortes de particules dans le milieu d'incubation, ce qui confère au dosage de très fortes précisions, impossibles à atteindre en turbidimétrie (opacimétrie) visible ou proche infra-rouge. A titre d'exemple, le tableau I suivant donne les densités optiques obtenues par lecture spectrophotométrique d'absorption ultra-violet-visible du résultat issu de l'agglutination des microsphères non colorées de polystyrène-anticorps de 0,8 micromètre de diamètre, et ce pour deux concentrations très basses de l'antigène correspondant.

**TABLEAU I**

| Concentration initiale en antigène | 10 ng/ml | 1 µg/ml |
|---|---|---|
| Densité optique. Absorption UV-visible à 380 nm | 1,3 | 0,5 |

La longueur d'onde de 380 nm, correspond au maximum de l'absorption de la lumière ultra-violet-visible par des microparticules monomères non-agglutinées,
  - le degré de précision permis par le mode de lecture selon l'invention, permet outre l'utilisation de n'importe quel spectrophotomètre, l'emploi de cuves de lecture d'un modèle courant (1 x 1 cm).

Les résultats fournis dans le tableau précédent correspondent à une mesure effectuée au moyen d'une cuve de lecture traditionnelle de 1 cm de chemin optique. Il faudrait un chemin optique de 4 centimètres, difficile à réaliser sur le plan pratique, pour obtenir la même précision en turbidimétrie.

Le dispositif de mise en oeuvre du procédé décrit précédemment comprend, outre un spectrophotomètre d'absorption de modèle courant, un boitier compartimenté et un moyen apte à imposer un mouvement vibratoire à la solution contenant les microsphères.

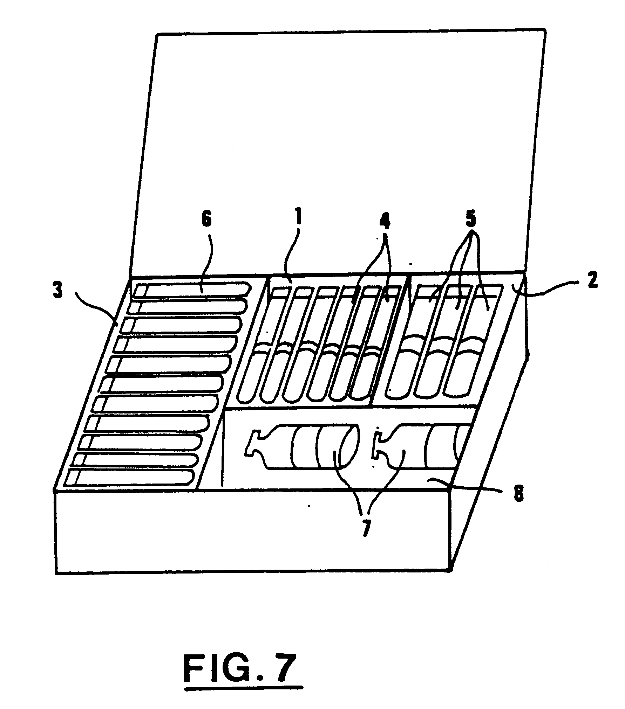

Le boitier compartimenté, présenté en figure 7, en forme de parallélépipède rectangle a pour dimensions 13 x 16 X 4 cm et est réalisé en matière plastique ou en carton rigide. Il présente quatre compartiments :
  - le premier compartiment (1) renferme une pluralité de tubes à hémolyse (4) horizontaux et parallèles, fermés et étiquetés (portant la concentration d'antigènes), et contenant 2 à 4 gammes étalon sous forme lyophilisée,
  - le deuxième compartiment (2) renferme également une plur alité de tubes à hémolyse (5), mais de taille plus importante, horizontaux et parallèles, et contenant le réactif prêt à l'emploi en l'occurence les anticorps greffés sur des microsphères, baignant dans un diluant approprié. En effet, le diluant doit assurer la conservation des anticorps, leur stablité dans le temps et leur bonne intégrité. Ces tubes sont au nombre de quatre par gamme étalon, et renferment 2,5 millilitres de réactif, la concentration en poids des microparticules dans le réactif est avantageusement compris entre 0,1 et 10 pourcent et plus particulièrement entre 1 et 5 pourcent,
  - le troisième compartiment (3) comporte une pluralité de tubes fermés (6) de petit diamètre, renfermant un échantillon de la solution de dilution de la gamme étalon, sous forme lyophilisée. La quantité de solution de dilution est de façon avantageuse, la quantité nécessaire à la dilution d'un échantillon biologique, par exemple la quantité nécessaire pour effectuer la dilution au dixième d'un échantillon biologique qui a déjà été dilué par de l'eau distillée. Une quantité de 0,45 ml ou 0,9 ml convient par exemple très bien,
  - le quatrième compartiment (8) comporte au moins un flacon (7) de concentré du liquide de dilution.

Le moyen apte à assurer le mouvement périodique de haute fréquence et de faible amplitude des microsphères comprend avantageusement un moteur solidaire d'un axe vertical actionnant une came. Cette came communique à un plateau, lui-même solidaire d'un support de tubes parallèles, un mouvement périodique de haute fréquence et de faible amplitude, et de trajectoire circulaire. Ledit plateau de forme générale allongée, reste toujours parallèle à sa direction originale. Le moyen apte à assurer le mouvement périodique comprend, en outre, un châssis sur lequel on règle l'affichage de la vitesse de rotation du moteur et la mise en marche. L'amplitude du mouvement est limitée dans le cas présent à 4 mm et la fréquence affichée est de 20 Hertz.

Ainsi, lorsque l'on désire procéder à un dosage en antigène d'une solution, par exemple au dosage de la β 2 micro-globuline dans un sérum d'insuffisant rénal, on prélève tout d'abord 50 microlitres du sérum à analyser auquel on ajoute 0,45 ml d'eau distillée. De cette solution ainsi réalisée, on prélève 50 microlitres que l'on met dans un des tubes (6) du compartiment (3) préalablement reconstitué avec 0,45 ml d'eau distillée. On procède à une homogénéisation classique. Puis, on prélève 50 microlitres de ce tube, on les mélange avec 50 microlitres du réactif prêt à l'emploi, c'est-à-dire de l'anticorps correspondant, greffés sur les microsphères et présent dans l'un des tubes (5) du compartiment (2), et qui a été préalablement homogénéisé. La concentration en poids des microsphères est avantageusement environ 1 pourcent. La nouvelle solution ainsi formée, est placée sur le support solidaire du plateau, déjà en mouvement. Elle subit ce mouvement périodique pendant une durée de dix minutes, la fréquence de vibration étant de 20 Hertz et l'amplitude de 4 mm. Cette phase d'agglutination étant réalisée, on procède à la phase de dilution. Le volume obtenu passe de 100 microlitres à 3,6 ml, c'est-à-dire au volume correspondant à la cellule d'analyse spectrophotométrique. On verse alors le contenu du tube dans la cellule de lecture de 1 X 1 cm et on procède à une analyse spectrophotométrique au maximum d'absorption dans l'ultra-violet-visible des microsphères non agglutinées, en l'occurence à 380 nanomètres, c'est-à-dire à la frontière du visible et de l'ultra-violet.

On peut également effectuer la lecture en plaçant directement les tubes à hémolyse dans le spectrophotomètre.

Au préalable, l'une des gammes étalon présente dans le compartiment (1) du boitier, a été utilisée afin de tracer la droite représentative de la relation entre la densité optique et le logarithme de la concentration en antigène. Une fois la densité optique de la solution obtenue déterminée, on la compare à la courbe étalon et l'on en déduit de façon extrêmement rigoureuse la concentration en antigènes du sérum initial.

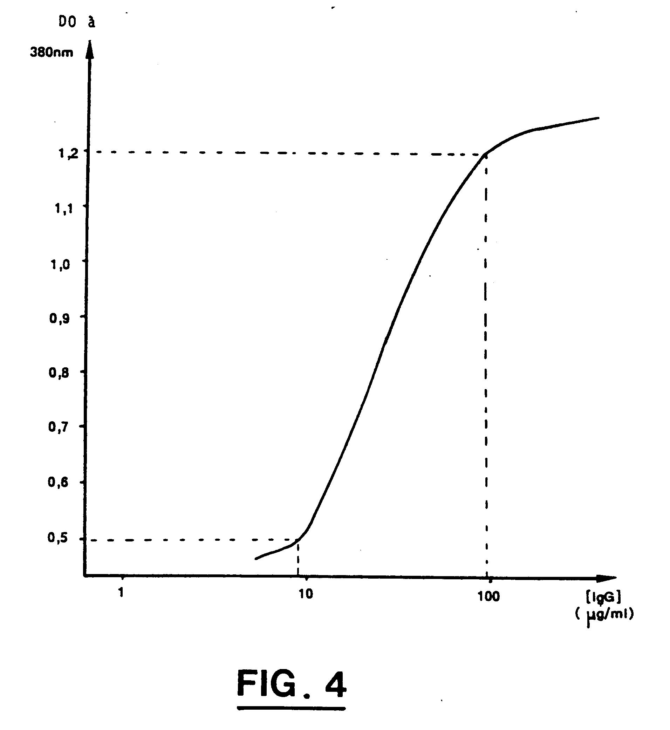

La figure 4 est une illustration d'un dosage d'Immuno-gamma-globulines G humaines au moyen du procédé et du dispositif conforme à l'invention. Dans cet exemple, c'est la partie droite par rapport au point d'équivalence de la courbe de la figure 1 qui est exploitée. Le coefficient de corrélation avec la néphélémétrie laser obtenu pour ce dosage sur cent sérums et liquides céphalorachidiens étant voisin de 1.

Le tableau II suivant est un tableau comparatif entre le procédé selon l'invention et un radio-immuno essai pour le dosage de la $\beta$ 2 microglobuline humaine dans des sérums normaux, d'insuffisants rénaux, et dans des liquides de dialyse.

**TABLEAU II**

Concentration en $\beta 2$ micro-globuline en $\mu$ g/ml

| | | | | | | | | |
|---|---|---|---|---|---|---|---|---|
| procédé selon l'invention | 11,6 | 5,7 | 0,7 | 2,97 | 2,24 | 2,13 | 2,71 | 2,23 |
| Radio-immuno essai | 13,2 | 6,7 | 0,41 | 2,06 | 2,12 | 2,05 | 2,60 | 2,30 |

De la présente invention, outre les avantages précédemment soulignés, se dégagent d'autres avantages parmi lesquels on peut citer :

- le dosage possible in situ, c'est-à-dire au lit même du malade, chose qui était rendue très difficile jusqu'à présent du fait de l'importance de l'appareillage à mettre en oeuvre,
- une très grande rapidité de mise en oeuvre et de détermination des résultats et ce, avec une très grande fiabilité, ce qui rend possible ce procédé en biochimie ou en pharmacologie d'urgence, et dans le suivi des greffes,
- l'analyse possible dans toute la zone des températures ambiantes habituelles.

Cette invention est donc particulièrement adaptée à de nombreux domaines de recherches et de la médecine, en particulier en cancérologie, hormonologie, endocrinologie, biochimie clinique, toxicologie et microbiologie.

D'autre part, elle présente un grand intérêt dans le domaine du contrôle pyrétogène de l'industrie pharmaceutique, et dans celui de l'hémodialyse.

**Revendications**

1. Procédé de dosage, d'une substance d'intérêt clinique réactive immunologiquement par d'autres substances du même type (dosage d'un antigène par un anticorps et réciproquement), du type selon lequel :
   - on greffe préalablement une substance active immunologiquement, jouant le rôle de réactif doseur sur des microparticules naturelles ou synthétiques,
   - on agglutine lesdites microparticules par la substance à doser d'intérêt clinique réactive immunologiquement,
   - on mesure par lecture le résultat issu de la réaction d'agglutination, que l'on compare à une gamme étalon,
      caractérisé en ce que la phase de lecture du résultat s'effectue :
   - tout d'abord, par détermination du maximum d'absorption par les particules de la lumière ultra-violet-visible d'une suspension en milieu liquide de microparticules non agglutinées, la détermination étant effectuée en faisant le spectre d'absorption ultra-violet-visible de ladite suspension, la longueur d'onde correspondant au maximum d'absorption étant dénommée λ**max,**
   - puis par lecture de résultat, par spectrophotométrie d'absorption ultra-violet -visible, en se plaçant au voisinage de ladite longueur d'onde λ**max** précédemment déterminée, après la réaction d'agglutination immunologique et une dilution du milieu d'incubation effectuée de manière connue.

2. Procédé de dosage selon la revendication 1 caractérisé en ce que les microparticules aptes à la lecture spectrophotométrique d'absorption ultra-violet-visible, sont des microparticules naturelles ou synthétiques de toute nature absorbante.

3. Procédé de dosage selon l'une quelconque des revendications 1 et 2, caractérisé en ce que les microparticules sont constituées par des polymères synthétiques choisis parmi le vinyltoluène, l'acrylonitrile, le styrène-butadiène, les esters acryliques, le vinylbutadiène, le polystyrène, les dérivés acrylonitrile-acide acrylique.

4. Procédé de dosage selon l'une quelconque des revendications 1 à 3, caractérisé en ce que les microparticules sont sous forme de microsphères.

5. Procédé de dosage selon la revendication 4, caractérisé en ce que les microsphères sont calibrées et ont un diamètre compris entre 0,01 et 5 micromètres.

6. Procédé de dosage selon l'une quelconque des revendications 1 à 5, caractérisé en ce que les microparticules sont non colorées.

7. Procédé de dosage selon l'une quelconque des revendications 1 à 5, caractérisé en ce que les microparticules sont colorées, la lecture du résultat par spectrophotométrie d'absorption ultra-violet-visible étant efffectuée, soit à la longueur d'onde correspondant à l'absorption maximum de la matière constitutive de la microparticule non agglutinée (λ**max**), soit à la longueur d'onde correspondant à l'absorption maximum de la couleur de la microparticule considérée.

8. Procédé de dosage selon la revendication 7, caractérisé en ce que la couleur des microparticules est choisie de manière à obtenir un effet de synergie entre le maximum d'absorption de la matière constitutive de la particule et le maximum d'absorption de la couleur de la particule.

9. Procédé de dosage selon l'une quelconque des revendications 1 à 8, caractérisé en ce que la suspension des microparticules est une suspension aqueuse.

10. Procédé de dosage selon la revendication 1, caractérisé en ce que la phase d'agglutination s'effectue en soumettant lesdites microparticules à un mouvement périodique de fréquence comprise entre 4 et 40 Hertz et d'amplitude variant de quelques millimètres à quelques centimètres.

11. Procédé de dosage selon la revendication 10, caractérisé en ce que la trajectoire du mouvement période est circulaire, rectiligne ou ovalisée.

12. Procédé de dosage selon l'une des revendications 10 et 11, caractérisé en ce que la phase d'agglutination s'effectue à température ambiante.

13. Procédé de dosage selon l'une des revendications 10 à 12, caractérisé en ce que la durée du mouvement périodique imposée aux microparticules durant la phase d'agglutination est fonction de la fréquence, de la trajectoire, de l'amplitude du mouvement et est de l'ordre de 10 minutes, pour une fréquence de 20 Hertz et un mouvement circulaire de 4 mm de diamètre.

**Claims**

1. Method of assaying an immunologically reactive substance of clinical interest using other substances of the same type (assay of an antigen using an antibody and vice versa) of the sort wherein :
   - an immunologically active substance as assaying reactant is initially attached to natural or synthetic microparticles,
   - said microparticles are agglutinated using the immunologically reactive substance of clinical interest to be assayed,
   - the result of the agglutination reaction is read off and is compared to a standard scale, characterised in that the reading off of the result is done :
   - firstly by determination of the absorption maximum in ultra-violet-visible light of the particles of a suspension of non-agglutinated microparticles in liquid medium, the determination being carried out while establishing the ultra-violet-visible absorption spectrum of said suspension, the wavelength corresponding to the absorption maximum being denoted λmax,
   - then, after the immunological agglutination reaction and having diluted the incubation medium by known means, by reading the ultra-violet-visible absorption spectrophotometry result in a wavelength region near the λmax previously determined.

2. Assay method according to claim 1, characterised in that the microparticles susceptible of being read by ultra-violet-visible absorption spectrophotometry are absorbent natural or synthetic microparticles of any kind.

3. Assay method according to claim 1 or 2, characterised in that the microparticles are made up of synthetic polymers chosen among vinyltoluene, acrylonitrile, styrene-butadiene, acrylic esters, vinylbutadiene, polystyrene and acrylonitrile-acrylic acid derivatives.

4. Assay method according to any of claims 1 to 3, characterised in that the microparticles are in the form of microspheres.

5. Assay method according to claim 4, characterised in that the microspheres are graded and have a diameter between 0.01 and 5 micrometres.

6. Assay method according to any of claims 1 to 5, characterised in that the microparticles are non-coloured.

7. Assay method according to any of claims 1 to 5, characterised in that the microparticles are coloured, the reading off of the ultra-violet-visible absorption spectrophotometry result being carried out, either at the wavelength corresponding to the absorption maximum of the material making up the non-agglutinated microparticle (λmax), or at the wavelength corresponding to the absorption maximum of the colour of the microparticle in question.

8. Assay method according to claim 7, characterised in that the colour of the microparticles is chosen in order to obtain a synergistic effect between the absorption maximum of the material making up the particle and the absorption maximum of the colour of the particle.

9. Assay method according to any of claims 1 to 8, characterised in that the microparticle suspension is an aqueous suspension.

10. Assay method according to claim 1, characterised in that the agglutination step is carried out by subjecting said microparticles to a periodic movement of frequency between 4 and 40 Hertz and of amplitude varying between a few millimetres and a few centimetres.

**11.** Assay method according to claim 10, characterised in that the trajectory of the periodic movement is circular, rectilinear or oval.

**12.** Assay method according to claim 10 or 11, characterised in that the agglutination step is carried out at ambient temperature.

**13.** Assay method according to any of claims 10 to 13, characterised in that the duration of the periodic movement to which the microparticles are subjected during the agglutination step is a function of the frequency, of the trajectory and of the amplitude of the movement and is of the order of 10 minutes for a frequency of 20 Hertz and for a circular movement of 4 millimetres diameter.

**Patentansprüche**

**1.** Verfahren zur Dosierung einer klinisch interessanten Substanz, die immunologisch reaktiv gegenüber anderen Substanzen desselben Typs ist (Dosieren eines Antigens mittels eines Antikörpers und umgekehrt), des Typs, gemaß dem man
- zuerst eine immunologisch aktive Substanz, die die Rolle eines Dosier-Reagenz spielt, auf natürliche oder synthetische Mikro-Teilchen aufbringt;
- die Mikro-Teilchen mittels der klinisch interessanten immunologisch reaktiven Substanz zum Dosieren agglutiniert;
- durch Ablesen das aus der Agglutinationsreaktion hervorgegangene Ergebnis abschätzt, welches man mit einer Eichskala vergleicht, dadurch gekennzeichnet, daß sich die Phase der Ablesung des Ergebnisses in der Weise vollzieht, daß man
- zu allererst das Absorptionsmaximum der Teilchen bei ultraviolettsichtbarem Licht für eine Suspension der nicht agglutinierten Mikro-Teilchen in flüssigem Milieu bestimmt, wobei die Bestimmung in der Weise durchgeführt wird, daß man das ultraviolett-sichtbare Absorptionsspektrum der Suspension aufnimmt, wobei die Wellenlänge, die dem Absorptionsmaximum entspricht, mit $\lambda_{max}$ bezeichnet wird;
- danach das Ergebnis mittels Absorptionsspektrophotometrie im ultraviolettsichtbaren Bereich nach der immunologischen Agglutinationsreaktion und einer Verdünnung des Inkubationsmediums, die in an sich bekannter Weise bewirktwird, abliest, wobei man in der Nähe der wie vorstehend beschrieben bestimmten Wellenlänge $\lambda_{max}$ bleibt.

**2.** Verfahren zur Dosierung nach Anspruch 1, dadurch gekennzeichnet, daß die Mikro-Teilchen, die zur spektrophotometrischen Ablesung der Absorption im ultraviolett-sichtbaren Bereich geeignet sind, natürliche oder synthetische Mikro-Teilchen beliebiger absorbierender Natur sind.

**3.** Verfahren zur Dosierung nach irgendeinem der Ansprüche 1 und 2, dadurch gekennzeichnet, daß die Mikro-Teilchen aus synthetischen Polymeren bestehen, die gewählt sind unter Vinyltoluol, Acrylnitril, Styrolbutadien, Acrylestern, Vinylbutadien, Polystyrol und Acrylnitril-Acrylsäurederivaten.

**4.** Verfahren zur Dosierung nach irgendeinem der Ansprüche 1 bis 3, dadurch gekennzeichnet, daß die Mikro-Teilchen in Form von Mikro-Kügelchen vorliegen.

**5.** Verfahren zur Dosierung nach Anspruch 4, dadurch gekennzeichnet, daß die Mikro-Kügelchen sortiert sind und einen Durchmesser zwischen 0,01 und 5 $\mu$m einschließlich aufweisen.

**6.** Verfahren zur Dosierung nach irgendeinem der Ansprüche 1 bis 5, dadurch gekennzeichnet, daß die Mikro-Teilchen nicht gefärbt sind.

**7.** Verfahren zur Dosierung nach irgendeinem der Ansprüche 1 bis 5, dadurch gekennzeichnet, daß die Mikro-Teilchen gefärbt sind, wobei die Ablesung des Ergebnisses durch Absorptionsspektrophotometrie im ultraviolett-sichtbaren Bereich entweder über die Wellenlänge, die dem Absorptionsmaximum des Materials entspricht, aus dem das nicht agglutinierte Mikro-Teilchen besteht ($\lambda_{max}$),oder über die Wellenlänge, die dem Absorptionsmaximum der Farbe des betrachteten Mikro-Teilchens entspricht, erfolgt.

14

**8.** Verfahren zur Dosierung nach Anspruch 7, dadurch gekennzeichnet, daß die Farbe der Mikro-Teilchen in der Weise gewählt wird, daß man einen Synergieeffekt zwischen dem Absorptionsmaximum des Materials, aus dem das Teilchen besteht, und dem Absorptionsmaximum der Farbe des Teilchens erhält.

**9.** Verfahren zur Dosierung nach irgendeinem der Ansprüche 1 bis 8, dadurch gekennzeichnet, daß die Suspension der Mikro-Teilchen eine wässrige Suspension ist.

**10.** Verfahren zur Dosierung nach Anspruch 1, dadurch gekennzeichnet, daß sich die Agglutinationsphase in der Weise vollzieht, daß man die Mikro-Teilchen einer periodischen Bewegung mit einer Frequenz zwischen 4 und 40 Hertz einschließlich und einer Amplitude unterwirft, die von einigen Millimetern bis zu einigen Zentimetern wechselt.

**11.** Verfahren zur Dosierung nach Anspruch 10, dadurch gekennzeichnet, daß der Verlauf der periodischen Bewegung kreisförmig, geradlinig oder unrund ist.

**12.** Verfahren zur Dosierung nach irgendeinem der Ansprüche 10 und 11, dadurch gekennzeichnet, daß sich die Agglutinationsphase bei Umgebungstemperatur vollzieht.

**13.** Verfahren zur Dosierung nach irgendeinem der Ansprüche 10 bis 12, dadurch gekennzeichnet, daß die Dauer der periodischen Bewegung, die den Mikro-Teilchen während der Agglutinationsphase auferlegt wird, eine Funktion der Frequenz, des Verlaufs und der Amplitude der Bewegung ist und für eine Frequenz von 20 Hertz und eine Bewegung in einem Kreis von 4 mm Durchmesser in der Größenordnung von 10 Minuten liegt.

FIG . 1

FIG . 2

FIG. 3

**FIG . 4**

FIG. 5

**FIG.6**

# FIG. 7